# EUROPEAN PATENT APPLICATION

(11) **EP 4 361 775 A1**
(43) Date of publication of application: **01.05.2024**
(21) Application number: 23206037.6
(22) Date of filing: 26.10.2023
(51) Int. Cl.: G06F 3/01, G16H 40/63, G16H 20/30, G16H 20/70

(54) **EXTENDED REALITY-BASED SYSTEM AND RELATED METHODS FOR TRAINING INDIVIDUALS IN SOCIAL MIRRORING SKILLS**

(30) Priority: 26.10.2022 US 202263419567 P
(71) Applicant: Vizmoo LLC, Wynnewood, PA 19096 (US)
(72) Inventor: Stauffer, Michael, Wynnewood, 19096 (US); DeHaven, Karen, Sellersville, 18960 (US)
(74) Representative: Marks & Clerk LLP

(57) **Abstract**

This disclosure pertains to computer-assisted methods, apparatus, and systems for treating individuals having neuroatypical conditions, such as Autism Spectrum Disorder (ASD), Attention Deficit Disorder (ADD), and Attention Deficit Hyperactivity Disorder (ADHD), to improve such individuals' ability to learn mirroring skills using virtual reality interfaces that cause the student to learn and practice such skills in the form of game play.

## Description

### FIELD OF THE INVENTION

This disclosure pertains to computer-assisted methods, apparatus, and systems for treating individuals having neuroatypical conditions, such as Autism Spectrum Disorder (ASD), Attention Deficit Disorder (ADD), and Attention Deficit Hyperactivity Disorder (ADHD), to improve such individuals' ability to learn mirroring skills.

### BACKGROUND

One of the fundamental ways that children (as well as adults) learn social skills is by mirroring the behaviors of others. For instance, much of human social interaction involves mirroring the body language or other social cues of another person with which an individual is socially interacting. Starting in infancy, mirroring is considered a necessary skill by healthcare specialists for the successful development of social communication skills in childhood and adulthood.

For many neuroatypical people, mirroring is a very difficult behavior to learn, which leads to failure to develop adequate social communication skills. Autism Spectrum Disorder (ASD) is a common neurodevelopmental disorder among children, and it often manifests as a neuroatypicality with regard to the skill of mirroring. ASD may manifest itself as impaired communication and social interaction skills. These impairments usually lead to deficits in social learning via mirroring skills, such as lessened ability to understand social cues, diminished attunement to teachers and/or other students, and difficulty initiating or responding to social interactions. Reduced ability to learn via mirroring may also be an aspect of other neuroatypical conditions such as Attention Deficit Disorder (ADD) and Attention Deficit Hyperactivity Disorder (ADHD).

Accordingly, individuals who are considered neuroatypical in the skill of social mirroring often receive social mirroring training. Such training commonly involves asking the individual being treated to copy (or mirror) the motions or other actions of another individual, e.g., a therapist. Such training is very burdensome because it essentially requires a human trainer with undivided attention to individually train another person for a lengthy continuous period of time, and commonly requires a large number of such sessions over many months. Furthermore, individuals that are neuroatypical with regard to the skill of social mirroring are, almost by definition, highly adverse to the type of face-to-face social interaction with another individual that is inherent in the very nature of social mirroring training.

### SUMMARY

In an embodiment, a computer-readable device comprises non-transitory instructions, which, when executed by a processor, cause the processor to perform the operations of generating an extended reality user interface (XRUI), tracking movement of at least one body part of a user in space, generating in the XRUI a follow marker, the follow marker comprising a representation of the movement of the tracked user's body part, generating in the XRUI a movement indicator, the movement indicator comprising a representation of a curvilinear path, wherein the path is depicted over time as moving toward a predetermined plane within the XRUI, calculating a distance in the XRUI between the follow marker and the movement indicator in the predetermined plane over time, and determining a follow success value indicative of the ability of the user to track the movement indicator with the follow marker. The operations may further comprise one or more of: generating in the XRUI an avatar of another being; gradually changing an appearance of the avatar; gradually changing a location of the avatar within the XRUI from a position that is off to a side of the user to a position in front of the user; gradually varying the movement indicator so as to increase a level of skill required of the user to follow the movement indicator; generating in the XRUI a supplemental movement indicator, the supplemental movement indicator comprising a mirror image of the movement indicator moving toward the avatar, and controlling a body part of the avatar to track the supplemental movement indicator; and including an audio track within the XRUI. Generating the movement indicator may comprise generating multiple movement indicators simultaneously and generating the follow marker may comprise generating multiple follow markers simultaneously. The audio track may comprise music that is synchronized to the movement indicator. The movement indicator may be generated so as to appear to be emanating from a body part of the avatar.

In another embodiment, an apparatus for training a user in social mirroring skills comprises an extended reality render module configured to generate an XRUI, the XRUI comprising a movement indicator, the movement indicator comprising a representation of a curvilinear path, wherein the path is depicted over time as moving toward a predetermined plane within the XRUI, a tracking module configured to track movement of a sensor in space, a motion module configured to generate in the XRUI a follow marker, the follow marker comprising a representation of the movement of the sensor in space, and a data capture/scoring module configured to calculate a distance in the XRUI between the follow marker and the movement indicator in the predetermined plane over time and determine a follow success value indicative of the accuracy with which the follow marker tracked the movement indicator. The extended reality render module may further display a score based on the follow success value and displays the score within the XRUI. The extended reality render module may generate the movement indicator by using the tracking module to track a sensor in space and generate the movement indicator by emulating the tracked movement of the sensor. The extended reality render module may further scale the tracked movement of the sensor to generate the movement indicator.

In yet another embodiment, a method of training users in social mirroring skills comprising generating an extended reality user interface (XRUI), tracking movement of at least one body part of a user in space; generating in the XRUI a follow marker, the follow marker comprising a representation of the movement of the tracked user's body part, generating in the XRUI a movement indicator, the movement indicator comprising a representation of a curvilinear path, wherein the path is depicted over time as moving toward a predetermined plane within the XRUI, calculating a distance in the XRUI between the follow marker and the movement indicator in the predetermined plane over time, and determining a follow success value indicative of the ability of the user to track the movement indicator with the follow marker. The method may further comprise one or more of: generating in the XRUI an avatar of another being; gradually changing an appearance of the avatar from fanciful to realistic; gradually changing a location of the avatar within the XRUI from a position that is off to a side of the user to a position in front of the user; gradually varying the movement indicator so as to increase a level of skill required of the user to follow the movement indicator; and generating in the XRUI a supplemental movement indicator, the supplemental movement indicator comprising a mirror image of the movement indicator moving toward the avatar, and controlling a body part of the avatar to track the supplemental movement indicator.

### BRIEF DESCRIPTION OF THE DRAWINGS

A more detailed understanding may be had from the detailed description below, given by way of example in conjunction with the drawings appended hereto. Figures in such drawings, like the detailed description, are exemplary. As such, the Figures and the detailed description are not to be considered limiting, and other equally effective examples are possible and likely. Furthermore, like reference numerals ("ref.") in the Figures ("FIGS.") indicate like elements.
FIG. 1 is a diagram illustrating a semi-conceptualized graphical user interface for training in solo mode shown in third-person view in accordance with embodiments;
FIG. 2 is a system diagram showing an exemplary network computing environment in which the present invention may be employed;
FIG. 3 is a schematic block diagram illustrating an exemplary and non-limiting embodiment of a computerized system in accordance with the present invention;
FIG. 4 is a diagram illustrating a semi-conceptualized graphical user interface for training in observer mode shown in third-person view in accordance with embodiments;
FIG. 5 is a diagram illustrating a semi-conceptualized graphical user interface for training in a first semi-mirroring mode shown in third-person view in accordance with embodiments;
FIG. 6A is a diagram illustrating a semi-conceptualized graphical user interface for training in a second semi-mirroring mode shown in third-person view in accordance with embodiments;
FIG. 6B is a diagram illustrating a semi-conceptualized graphical user interface for training in a third semi-mirroring mode shown in first-person view in accordance with embodiments;
FIG. 7A is a diagram illustrating a semi-conceptualized graphical user interface for training in a fourth semi-mirroring mode shown in third-person view in accordance with embodiments;
FIG. 7B is a diagram illustrating a semi-conceptualized graphical user interface for training in a fifth semi-mirroring mode shown in third-person view in accordance with embodiments;
FIG. 8 is a diagram illustrating a semi-conceptualized graphical user interface for training in direct-mirroring mode shown in third-person view in accordance with embodiments;
FIG. 9 is a diagram illustrating a semi-conceptualized graphical user interface for training in a first alternate mode shown in third-person view in accordance with embodiments;
FIG. 10 is a diagram illustrating a semi-conceptualized graphical user interface for training in a second alternate mode shown in third-person view in accordance with embodiments;
FIG. 11 is a diagram illustrating a semi-conceptualized graphical user interface for training in a third alternate mode shown in third-person view in accordance with embodiments; and
FIG. 12 is a flowchart illustrating operation in accordance with an exemplary embodiment.

### DETAILED DESCRIPTION

In the following detailed description, numerous specific details are set forth to provide a thorough understanding of embodiments and/or examples disclosed herein. However, it will be understood that such embodiments and examples may be practiced without some or all of the specific details set forth herein. In other instances, well-known methods, procedures, components, and circuits have not been described in detail, so as not to obfuscate the following description. Further, embodiments and examples not specifically described herein may be practiced in lieu of, or in combination with, the embodiments and other examples described, disclosed, or otherwise provided explicitly, implicitly, and/or inherently (collectively "provided") herein.

Since individuals with social mirroring deficits find it challenging to socially interact with other individuals, a method and apparatus is desired for training such individuals in the skill of social mirroring that incrementally increases the degree of social interaction as the person learns mirroring skills, thereby becoming incrementally more comfortable with social interaction and social mirroring.

In embodiments, a person is trained in social mirroring via the use of an extended reality (XR) system comprising methods and apparatus that allow a therapist (or other trainer) to incrementally increase or decrease the degree of difficulty, degree of perceived social interaction, and degree of instruction/guidance as an individual improves his/her social mirroring skills via the training regimen.

The term extended reality (or XR) is used herein as a catch-all phrase to encompass virtual reality (VR), augmented reality (AR), mixed reality (MR), and variations thereof. VR broadly refers to methods and apparatus wherein an individual is completely immersed in a sensory experience with no input from the physical world. The immersion is commonly limited to the visual sense, to the visual and auditory senses, or to the visual, auditory, and touch senses. Thus, for instance, a VR experience typically would require the wearing of a headset that completely shields the wearer's eyes (and/or ears) from stimulus from the physical world and only allows the wearer to see what is projected via the VR headset (and/or hear what is produced by the headset`s headphones or speakers). As technology advances, however, it may become common to extend the experience to include additional senses, such as taste, smell, and touch. In order to interact with the virtual environment, the individual may further wear or carry transducers (typically gloves or handheld devices that are often called "controllers" in gaming parlance) that the VR apparatus can track in space (e.g., via motion sensing equipment, such as 3-axis gyroscopes, 3- axis accelerometers, and/or 3-axis electronic compasses, in the controllers and/or cameras pointed toward the controllers) that detect the movement of the controllers and then project some representation of that movement into the VR display scene. For instance, the individual may wear gloves that are tracked via the VR system and, within the VR scene, a pair of hands are shown with movement commensurate with the movement of the gloves (i.e., the person's hands). In other embodiments, cameras may track the individual's hands without the need for the individual to wear or hold any particular controller.

Augmented reality, on the other hand, allows at least some sensory stimulus from the physical world to be combined with virtual sensory stimulus provided by the headset. A typical configuration for an AR experience is a pair of eyeglasses that allow the wearer to see the physical world, but also projects some virtual elements onto the lenses of the glasses so that the wearer sees both the physical world as well as the virtual elements. Of course, the same mixing of sounds from both the physical world and sounds produced by headphones also may be a feature of an AR user interface.

Mixed reality is similar to AR, but wherein the virtual elements can interact with the real-life elements and the user can interact with the virtual elements.

Note that, as an alternative to the above-described embodiments of AR and MR, in which the user sees the real world directly through a pair of see-through glasses, in other embodiments, AR and MR systems may utilize a full headset that blocks out all direct visual (and/or audio) stimulus from the physical world, but captures such stimuli from the physical world via cameras and/or microphones, respectively, and then feeds those captured images or sounds into the headset via projection and/or speakers, respectively, to be combined with virtual stimuli.

Herein, the term gameplay level is used to refer to a collection of movement indicators that are presented to the individual being trained (hereinafter often referred to as the student) for the student to follow, possibly with accompanying music. This is the core gameplay of the experience. Herein, the word step is used to refer to an action taken to change the system in some way, either by the individual doing the training (hereinafter often referred to as the therapist) or algorithmically by the system, e.g., changing settings for features such as indicator time window, gameplay level, start/stop of gameplay, avatar appearance. Herein, the word mode is used to refer to a named state of the system in which one or more particular steps have been made/applied and are within a set of values that does not change. For example, in one mode, herein termed semi-mirroring mode, in which the student is instructed to mirror an avatar's motions (explained in greater detail below), an indicator time window setting has a certain set of values, and the bot avatar position has a certain set of values). However, in embodiments, other steps, like changing avatar appearance, can be made without changing mode.

In an embodiment, a computer-generated, 3-dimensional XR experience is created that the student experiences (e.g., sees and hears) that guides the student through increasing degrees of difficulty and social interaction (e.g., mirroring). The social mirroring training regimen may be presented in the form of gameplay such that the individual essentially considers the experience to be one of playing an entertaining game, rather than training or learning.

As will be described in more detail below, in one exemplary embodiment, the student wears an XR headset and holds or wears trackable sensors in or on his/her hands (or other body parts, as will be discussed further below). In a first form of use (herein sometimes referred to as solo gameplay), the system presents the student with an XR user interface (XRUI) comprising a visual pattern that the student has to follow with his/her hands (sometimes referred to herein as a movement path or movement indicator).

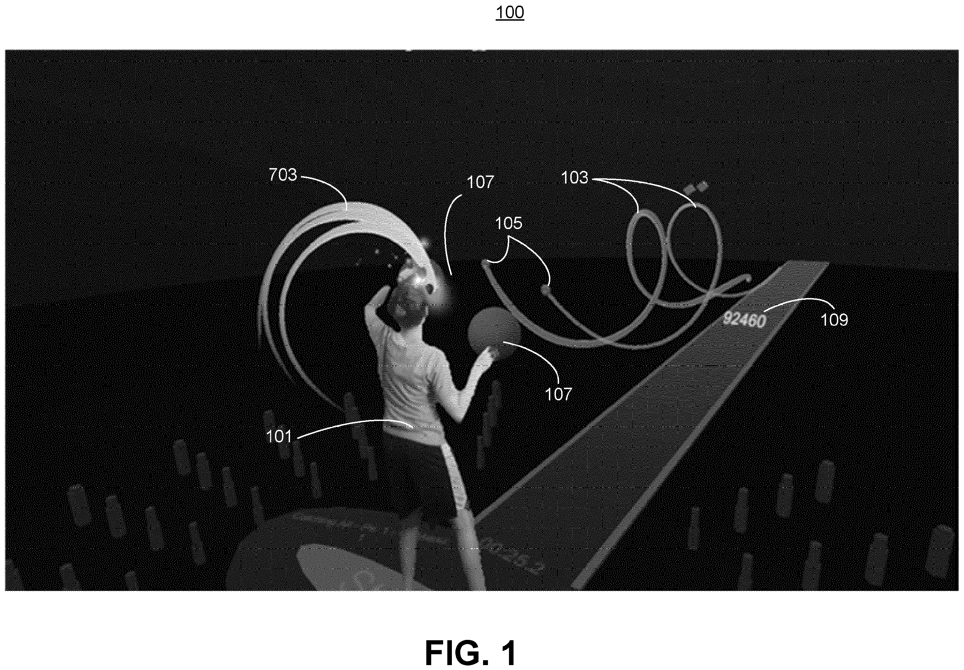

FIG. 1 shows an example of the concept. Thus, for instance, the student 101 may be presented with two movement indicators, e.g., curvilinear, flowing lines 103 moving toward him, wherein the goal is for the student to move his/her hands so that they track the paths as the paths reach the student's virtual hands within the XR display. The system may capture data as to the performance of the student (e.g., accuracy in following each movement indicator 103) and may even show a score 109 to the student during game play.

It should be understood that, for sake of explanation, FIG. 1 shows the student in the XR environment, which, in a preferred embodiment, is not how the virtual scene would actually be presented to the student. Rather, in order to most closely emulate real life, in a preferred embodiment, the VR display would show to the student only a virtual representation of the portions of the student's body parts that would be within that student's field of view or, even more preferably (although less realistic looking), just the body part(s) that are relevant to game play (e.g., those body parts that are being tracked by the XR system). This is because, just as in real life, one does not see one's whole self.

On the other hand, in certain embodiments, another person participating in the XR experience (e.g., the therapist) might be presented with a view like that of FIG. 1, in which the therapist sees the student's entire body (or a virtual representation of the student's body, i.e., an avatar). For instance, in a VR embodiment, the system may be configured to present to the therapist a computer-generated avatar corresponding to the student. On the other hand, in an AR or MR experience, the system might be configured so that the therapist sees the real student, such as shown in FIG. 1.

Furthermore, it should be understood that, while the embodiments discussed herein generally focus on using one's hands to track the virtual stimuli, it is equally possible, and, in fact, desirable, to perform the training using other body parts as well (e.g., feet, elbows, head, eyes, etc.). In some embodiments, part of the increasing difficulty of training may involve adding or altering body parts that must be used to track movement guides presenting in the VR display. In yet other embodiments, one also may be asked to copy or mirror facial expressions as part of more advanced gameplay.

In an embodiment, the hardware portion of the apparatus may comprise largely conventional XR hardware, such as a VR, AR, or MR audio/visual headset and one or more handheld trackable sensors (e.g., controllers). In more complex configurations, external cameras may be pointed at the student instead of or in addition to the trackable sensors to capture the movements of the student for purposes of emulating it in the XR display. In an embodiment, the software or firmware to cause the hardware to perform all of the functionality described herein may be provided as a plug in module to the headset, as software that is loadable onto to a computer that runs the hardware (which may be within the headset or external to it and connected to the headset via wires or wirelessly, e.g., via Wi-Fi or Bluetooth), or may be cloud-based software that controls the headset via a communication network wirelessly or via wires).

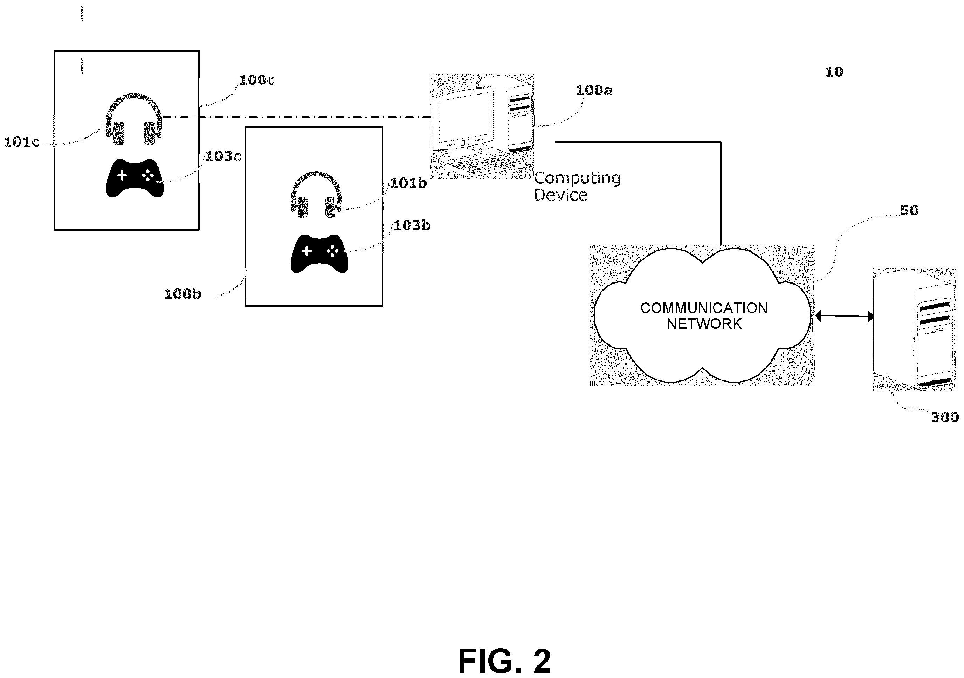

Fig. 2 is a system diagram showing an exemplary computing environment in which embodiments may be employed. As shown, the exemplary network environment 10 may include certain conventional computing hardware and software for communicating via a communication network 50, such as the Internet. FIG. 2 illustrates an example in which at least some of the software for generating the XR user interface is cloud-based. It also illustrates a configuration in which the XR rig 100b (which may comprise a headset 101b and one or more controllers 103b) is independent in the sense that it includes all of the hardware and software needed to render the XR user interface based on any data received via the network from, e.g., a cloud server 300. Finally, it further illustrates an alternate configuration in which XR rig 100c (which may comprise a headset 101c and one or more controllers 103c) is not independent, but rather is controlled by a separate computing device 100a which may be connected to 100c either wirelessly or via wired connection, and which may receive data from the server 300 via the network 50 to transmit to the XR rig 100c.

Computing device 100a may be, for example, a personal computer/PC, laptop computer, tablet computer, smartphone, or other computing system hardware, including computerized/networked communication hardware/software/functionality, such as computer-based servers, kiosks and the like, or other so-called "connected" communication devices having communication capabilities for communicating data via the network.

In accordance with certain embodiments, devices 100a, 100b, 100c are configured to store and execute one or more "apps" or other purpose-specific application software for rendering the XR user interfaces and enabling the other features discussed herein.

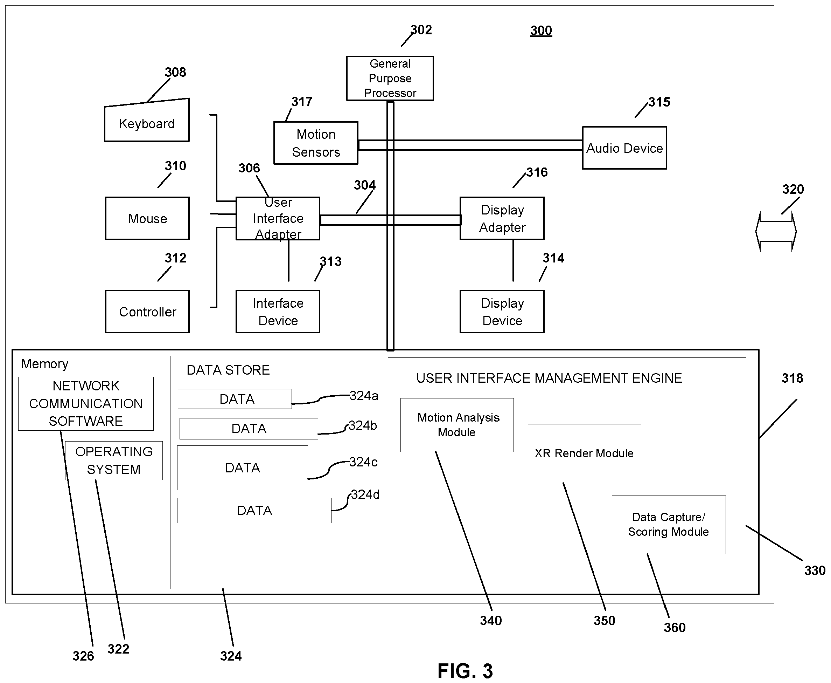

Hardware and software for enabling communication of data by such systems via such communications networks are well known in the art and beyond the scope of the present invention, and thus are not discussed in detail herein. FIG. 3 is a schematic block diagram showing the primary components of a system configuration in accordance with an exemplary embodiment, It should be understood that the individual components shown in FIG. 3 are representative of the components that might be found in any of the physical configurations mentioned or made obvious herein, such as (1) a self-contained XR rig in which all of the hardware as well as software for generating the XR user interface is stored in the rig itself without the need to receive any data in real-time via external sources (such as the network 50), (2) the combination of computer 100a and XR rig 100c connected to server 300 via a network shown in FIG. 2, (3) the independent controller 100b connected to server 300 shown in FIG. 2, and (4) a combination of a computer and an XR rig without the need to receive any data in real-time via external sources (such as a network). The only significant difference between the various configurations being that the various components might be contained within different physical devices (e.g., some components might be found in computer 100a, other components might be found in rig 100c, and yet other components might be found in server system 300).

The rig 300 is a special-purpose computer system that includes conventional computing hardware storing and executing both conventional software enabling operation of a general-purpose computing system, such as operating system software, network communications software, and specially-configured computer software for configuring the general purpose hardware as a special-purpose computer system for carrying out at least one method in accordance with the present invention. By way of example, the communications software may include conventional web server software, and the operating system software may include iOS, Android, Windows, and/or Linux software.

In some embodiments, the system 300 may, for example, execute, process, facilitate, and/or otherwise be associated with the embodiments described herein.

Accordingly, the exemplary system 300 includes a general-purpose processor, such as a microprocessor (CPU) 302 and a bus 304 employed to connect and enable communication between the processor 302 and the components of the XR interface hardware in accordance with known techniques. According to some embodiments, the processor 302 may be or include any type, quantity, and/or configuration of processor that is or becomes known. In some embodiments, the processor 302 may comprise multiple interconnected processors, microprocessors, and/or micro-engines. According to some embodiments, the processor 302 (and/or the system 300 and/or other components thereof) may be supplied power via a power supply (not shown), such as a battery, an Alternating Current (AC) source, a Direct Current (DC) source, an AC/DC adapter, solar cells, and/or an inertial generator.

The exemplary system 300 includes a user interface adapter 306, which connects the processor 302 via the bus 304 to one or more interface devices. In a system that includes a separate computing device, such as computing system 100a in FIG. 2, the interface devices might include keyboard 308 and mouse 310. The aforementioned handheld controller(s) or glove(s) also are interface devices 312. For example, the system might generate and display options and/or prompts within the XR display that a user can select or otherwise interface with using a controller. Other possible interface devices 313 include a touch-sensitive screen, a digitized entry pad, etc.

The bus 304 also connects one or more display devices 314, such as LCD screens, projectors, and a XR headset for rendering 3-D images to the user, to the processor 302 via a display adapter 316.

The bus 304 also connects to the processor one or more audio devices 315, such as speakers and microphones for rendering the audio aspects of the 3-D interface to the user.

The bus 304 also connects the processor 302 to a memory 318, which can include a hard drive, a solid-state drive, an optical drive, a diskette drive, a tape drive, etc. The memory 318 may comprise any appropriate information storage system that is or becomes known or available, including, but not limited to, units and/or combinations of magnetic storage systems (e.g., a hard disk drive), optical storage systems, and/or semiconductor memory systems, such as RAM systems, Read Only Memory (ROM) systems, Single Data Rate Random Access Memory (SDR-RAM), Double Data Rate Random Access Memory (DDR-RAM), and/or Programmable Read Only Memory (PROM).

The memory 318 may, according to some embodiments, store one or more software components. Any or all of the exemplary instructions and data types described herein, and other practicable types of data may be stored in any number, type, and/or configuration of memory systems that is or becomes known. The memory 318 may, for example, comprise one or more data tables or files, databases, table spaces, registers, and/or other storage structures. In some embodiments, multiple databases and/or storage structures (and/or multiple memory systems) may be utilized to store information associated with the system 300. According to some embodiments, the memory 318 may be incorporated into and/or otherwise coupled to the system 300 (e.g., as shown) or may simply be accessible to the system 300 (e.g., externally located and/or situated). The memory may store network communication software 326 and operating system software 322.

The rig 300 may communicate with other computers or networks of computers, for example, via a communications channel, network card, modem, or transceiver (collectively, "transceiver") 220. In some embodiments, the transceiver 220 may comprise any type or configuration of communication system that is or becomes known or practicable. The transceiver 220 may, for example, comprise a Network Interface Card (NIC), a telephonic system, a cellular network system, a router, a hub, a modem, and/or a communications port or cable. According to some embodiments, the transceiver 220 may also or alternatively be coupled to the processor 302. In some embodiments, the transceiver 220 may comprise an IR, RF, Bluetooth^{™}, Near-Field Communication (NFC), and/or Wi-Fi^{®} network system coupled to facilitate communications between the processor 302 and a network (not shown). The system 300 may be associated with such other computers in a local area network (LAN) or a wide area network (WAN), and may operate as a client in a client/server arrangement with another computer, etc. Such configurations, as well as the appropriate communications hardware and software, are known in the art.

The system 300 is specially configured in accordance with the present invention. Accordingly, as shown in Fig. 3, the system includes computer-readable, processor-executable instructions stored in the memory 318 for carrying out the methods described herein. Further, the memory 318 stores certain data, e.g., in one or more databases or other data stores 324 shown logically in Fig. 3 for illustrative purposes, without regard to any particular embodiment in one or more hardware or software components.

Further, as will be noted from Fig. 3, the system 300 includes, in accordance with the present invention, a User Interface Management Engine 330, shown schematically as stored in the memory 318, which includes a number of additional modules 340, 350, 360 for providing the special-purpose functionality for training individuals in mirroring, etc. as discussed in detail herein. These modules may include, for instance, a module 350 for producing and rendering the XR environment in the headset. These modules may include, for instance, a module 340 for analyzing the detected motion and operation of the headset and/or controllers in order to adjust the display and sound according to the student's head movement and controller movement and operation. These modules may include, for instance, a module 360 for capturing the data indicative of the student's performance and for generating a score that the student and/or therapist can use to evaluate how well the student is doing. These modules may be implemented primarily by specially-configured software including microprocessor-executable instructions stored in the memory 318 of the system 300. Optionally, other software may be stored in the memory 318 and and/or other data may be stored in the data store 324 or memory 318.

Although the terms "step", "block", "module", "engine", etc. might be used herein to connote different logical components of methods or systems employed and/or for ease of illustration, the terms should not be interpreted as implying any particular order among or between various steps herein disclosed unless and except when the order of individual steps is explicitly described, or be interpreted as implying any distinct structure separate and apart from other structures of the system.

Referring again to FIG. 1, the system generates and renders an XR environment (e.g., a graphical and/or auditory user interface) that includes one or more of the following user interface elements as described herein below.

The system may be configured to control the user interface (UI) to render one or more movement indicators of some kind (visual, auditory), e.g., flowing lines indicator 103, that guide the student to make particular movements by following the movement indicators. The flowing line movement indicators 103 flow/move toward the student and indicate to the student where the student's hands (or other body part, as the case may be) should be at the current moment via a movement target 105 at the near end of the flowing line movement indicator 103. For each movement indicator 103, the movement target 105 may be located in a plane in the virtual environment that is generally close to and in front of the student's expected location in the virtual environment, e.g., close to and in front of the follow markers 107. This plane, particularly if there are two or more motion indicators for the student to track, should be generally parallel to the student's expected sagittal plane in the virtual environment.

Each movement target 105 indicates where the student's corresponding body part should be located in space at the current moment. It is the primary target that the user is trying to follow with their follow marker 107. The 'follow marker' 107 is the XR representation of the student's body part (e.g., hand) that the student is using to try to follow the movement indicator. For example, a partially transparent sphere 107 may be centered on the body part, such that it moves with the student's real-world body part. The student's goal is to align each follow marker 107 with the appropriate movement target 105 of each movement indicator 103.

The movement indicator 103 also may indicate to the student where the student will need to move in the immediate future, i.e., the portions of the movement indicators 103 that are farther away from the movement target portion 105 of the movement indicator 103 (as seen from the student's perspective). There may be multiple movement indicators, each meant to be followed by a different body part, e.g., the user's hands, head, feet and/or other body parts, depending on the options available for the XR system, and the options chosen by the system operator.

The movement indicator 103 shows a rendering of a visible portion of a larger movement path. Particularly, a movement indicator 103 is the portion of an overall movement path that is represented in the XR environment at any given moment in time. The movement path controls the path that a movement indicator 103 follows and visualizes. It is the path that the student is being prompted to follow via the movement indicator 103 and movement target 105. At any given moment, there may be (1) a portion of the movement path that has already "passed" the user (and which may no longer be seen in the UI, but which had previously been represented by the movement indicator 103), (2) a portion of the movement path that is represented by the movement indicator 103, and (3) a portion of the movement path that is too far in the future to yet be displayed as part of the movement indicator 103). In other embodiments, the movement indicator may be configured to actually keep showing some portion of the movement path that has already "passed by" the student.

The movements of a movement indicator 103 are dictated by the corresponding movement path. As will be discussed in more detail below, there may be three types of movement paths, namely, a recorded path, a live path, and a procedural path.

A timeline controls the visual placement of movement indicator components, with the position of items at the current time always within reach of the student. In some embodiments, future times are farther away from and in front of the student, and past times are positioned behind the current time and ultimately behind the student. The movement target is always set to be visualized at the current time position.

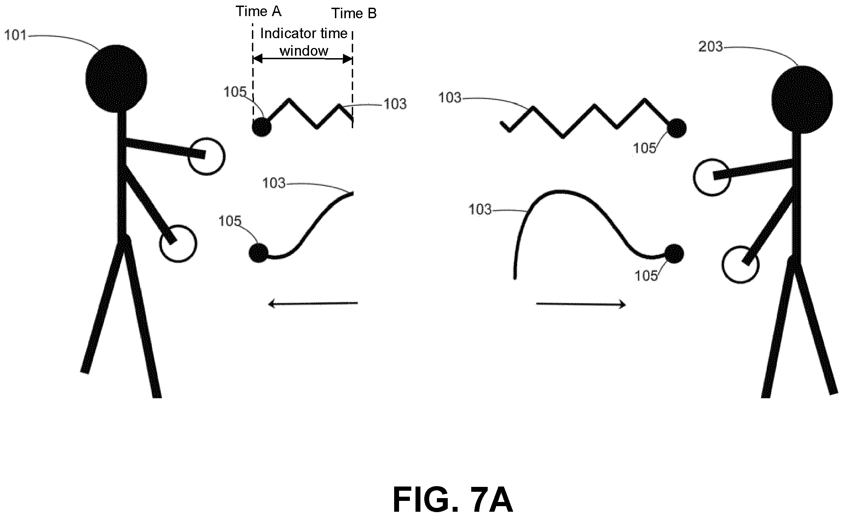

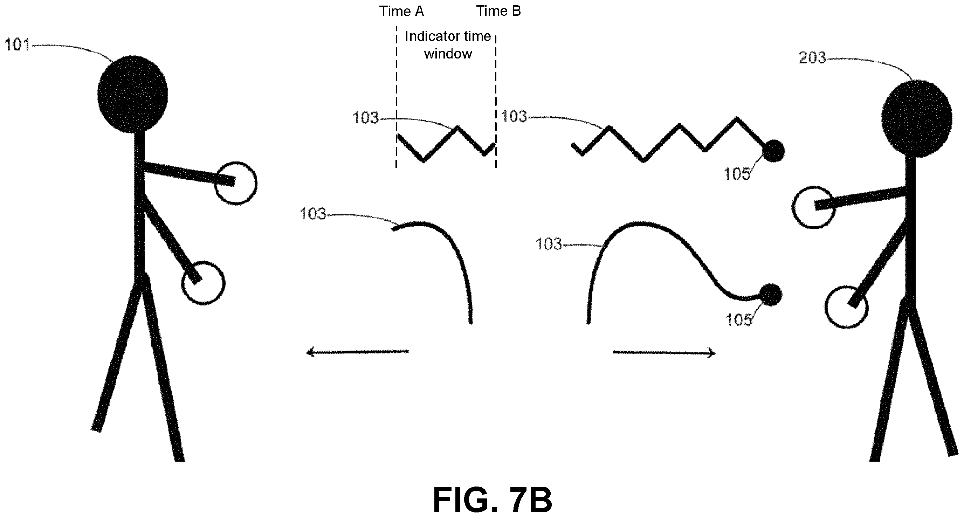

Each movement indicator 103 is partially defined in the software by an indicator time window. The indicator time window defines the duration of time spanning a time A to a time B, over which a movement indicator 103 supplies indication of where and how the student is expected to next move. It essentially dictates the length of the movement indicator 103 that trails the movement target 105. Reference may be made to Fig. 7A or Fig. 7B for an exemplary visual representation of the indicator time window. A shorter time window means less of the movement path's future movements are shown, and vice versa for a longer time window. The indicator time window's duration is calculated as B minus A, where Time A may be after, before, or equal to the current time, and Time B is always a time after (i.e., further in the future than) both Time A and the current time. The times A and B are initialized by the system or by the therapist, and the duration may be changed by the system or by the therapist. There may be two modes for changing times A and B, namely, default mode and inverted mode. In default mode, as the duration changes, Time A is always set to a past time or the current time, and Time B changes. In inverted mode, Time A starts at the current time, and as the duration changes, only Time A changes, by moving further into the future, and Time B does not change. The indicator time window may be set to zero, in which case there is nothing shown other than possibly the movement target 105.

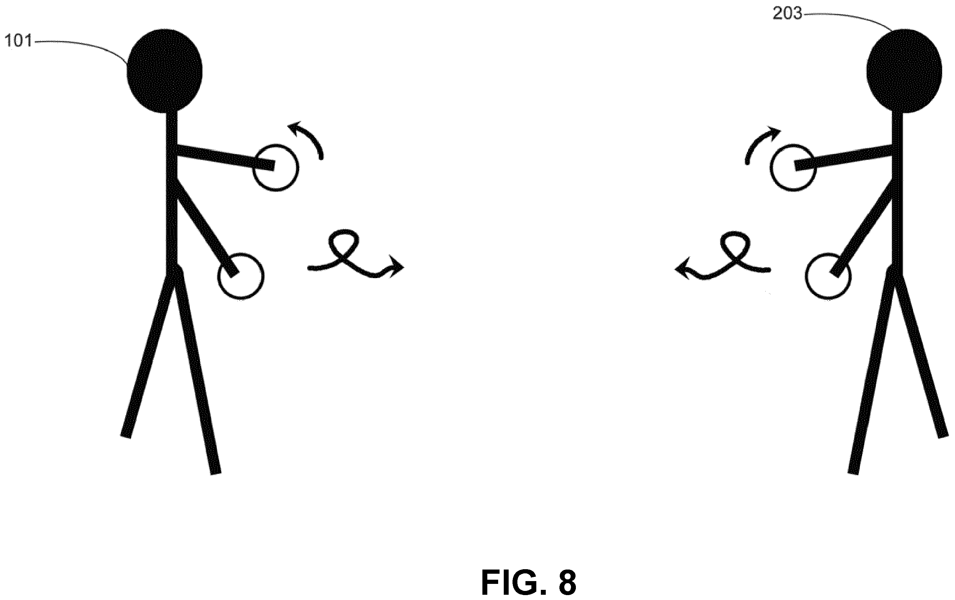

Thus, in default mode, the display is set to display to the student indications of future movements from the current moment forward, whereas, in inverted mode, the display is set to show the student future movements, but the student sees nothing closer to the follow marker other than possibly the movement target 105. As the indicator time window decreases in inverted mode, the closest portion of the movement path that the student is allowed to see moves further and further away into the future. The idea of inverted mode is that the student is motivated to start looking at the avatar's movements to be able to follow properly (in embodiments in which there is an avatar - not shown in FIG. 1, but see FIG. 8 for an example).

An indicator time window may be applied as a global value to all movement indicators, or to a subset of movement indicators, or individually to each or a single movement Indicator depending on the system settings and therapist's goals.

As previously mentioned, there may be at least three types of movement paths, namely, a recorded path, a live path, and a procedural path.

A recorded path is a type of movement path made any time before its use and loaded at runtime to control the path of the movement indicator 103. It may be preconfigured in the system, created by the therapist and saved, or created by a different person (and may be shared via an online portal created for sharing paths). The movement paths may be recorded within the system in a recording mode during which the path creator, typically listening to a music recording, moves and the system records their movements to generate movement paths.

Live paths are created on the fly (i.e., in real time) by a therapist for the student to follow. For instance, a therapist can control the movement indicators in real-time during the training via the therapist's own movements. The therapist's follow markers 107 may become aligned with the therapist's movement targets, or they may not be shown at all. For example, with the flowing lines type movement indicators 103 shown in FIG. 1, the lines 103 may emanate from the therapist's hands as they move, and flow towards the student for the student to follow. If music or other auditory input is included in the XR environment (as discussed in more detail below), when an indicator time window of non-zero length, N, is configured, the student's music playback time is delayed relative to that of the therapist by a period equal to the 'Time B' value of the indicator time window, such that the follow markers 107 of both participants are moving to the music playback for each participant with the same timing (if the student is following accurately). This last aspect is important for maintaining musical synchronization between the creation of the live path and the student's following thereof.

A procedural path is a movement path generated by the system algorithmically. It may use portions of recorded paths and modify them algorithmically, or it may generate the path completely algorithmically, or by some combination of the two methods. The parameters of the algorithms may be modified by the therapist to create paths of different difficulties and/or with different movement goals.

In an embodiment, the system may be configured to permit spatial scaling of all of the types of movement paths so that the movement paths will always be within the reach of the student, regardless of the student's size and/or physical limitations, via a process termed reach mapping. A human participant's reach mapping is created by the system through either a simple or more thorough procedure to determine how far they can reach with each body part within the surrounding space. A simple procedure measures participant's height (whether standing or sitting), shoulder height, and arm length. A more thorough procedure measures the participant's ability to reach all regions in the surrounding space, and accounts for possible limitations of movement of different body parts in different directions. For example, a participant may not be able to move their arms above their shoulder height. Once a 'reach mapping' has been generated, all Movement Paths may be scaled such that every part of the path will be reachable by the participant, and the details of the shape of the path may be preserved as much as possible. For example, a 6-foot tall, non-movement-restricted therapist may create a recorded path for hand movements, part of which involves sweeping arm movements that reach high overhead. If a 4' 6", non-movement-restricted child then attempts to follow this recorded path, all of the spatial locations within the path will be scaled to be within reach of the child, yet still lead to the same arm movements as the child follows the path, including the sweeping overhead movements. If, on the other hand, a child with one movement-restricted arm and one non-restricted arm attempts to follow the paths, the path for the movement-restricted arm will further be modified to always be within reach of the child, while maintaining as much as possible the motion dynamics of the original path.

In operation, over a series of sessions, the system will lead the student to improve social mirroring and social-emotional skills via gameplay and gradual desensitization to social challenges associated with mirroring behaviors. The training should result in improved real-life mirroring skills that are central to improved social-emotional skills and comfort, and thereby lead to increased participation and engagement in school, leading to improved learning outcomes. The system leverages characteristics of VR to create a controlled, immersive environment that can be tailored for a student's comfort.

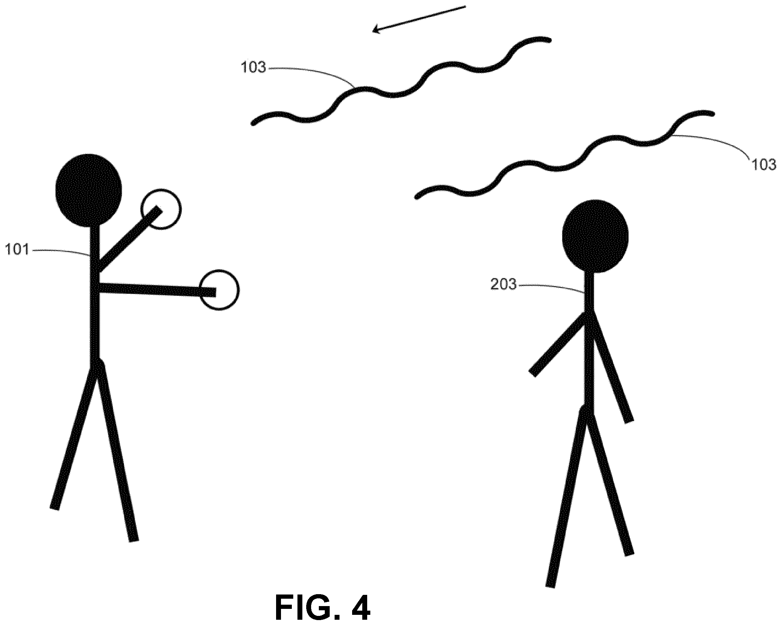

In one exemplary embodiment, the game play/training starts with the system generating a user interface for a non-mirroring "solo" mode of gameplay, such as was illustrated in FIG. 1, wherein the student tries to track the movement indicators 103 with his/her hands. This may be followed by a second mode of gameplay illustrated by FIG. 4 (sometimes referred to herein as the "observer" mode of gameplay). This mode essentially is the same as in the solo mode of gameplay, but with the system generating and displaying a non-threatening avatar 203 within the XR environment that is within the student's field of view but who is simply observing the student 101 playing the game. If the therapist is controlling this partner avatar 203, the therapist can also encourage the student via verbal and/or gestural feedback. The goal of this observer mode is to acclimate the student to the presence of the partner avatar. When the student and therapist are in the same physical room, the therapist may offer verbal encouragement either via audio connection in the XR headset or simply via regular speaking to the student in the room.

The system may at first configure the partner avatar 203 as a very non-realistic looking avatar, such as a cartoon character or anthropomorphized animal so that the student is more comfortable with the avatar than might be the case with a more realistic human-looking avatar. The system may be configured to allow the student to select the avatar from a plurality of options, which may even include options for customizing a selected base avatar.

As will be discussed in more detail below, the system may alter the partner avatar display over the course of the sessions and/or gameplay levels to become increasingly more human and realistic looking in order to slowly increase the student's comfort with the presence of another real person. In one embodiment, the system gradually modifies the avatar to look like the student's therapist or another trusted person in the student's life (e.g., a parent or sibling).

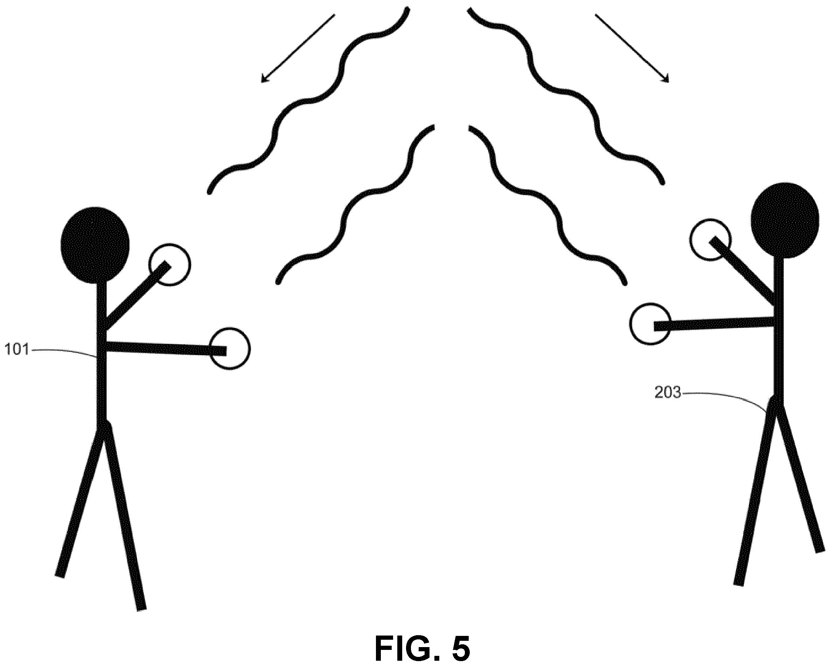

The observer mode of play may be followed with a "passive-mirroring" mode of gameplay followed by a "direct-mirroring" mode of gameplay. Each of those modes may comprise gameplay levels of increasing difficulty, increasing social interaction, and/or increasing degree of mirroring behavior required. For example, in a first phase of passive-mirroring gameplay, such as illustrated by FIG. 5, the avatar 203 in the scene may simply play the same game side-by-side with the student 101 wherein the system generates a supplemental movement indicator, the supplemental movement indicator being a mirror image of the student's movement indicator(s) and that moves toward the therapist avatar that the therapist avatar follows so that the student is not really mirroring the avatar but is simply playing the same game as the avatar. They are engaging in what therapists call parallel play, which may be viewed as a kind of side-by-side mirroring. However, the student is following his/her own set of movement indicators, and thus is not relying on watching the avatar to know what movements to make, and thereby is considered passive-mirroring.

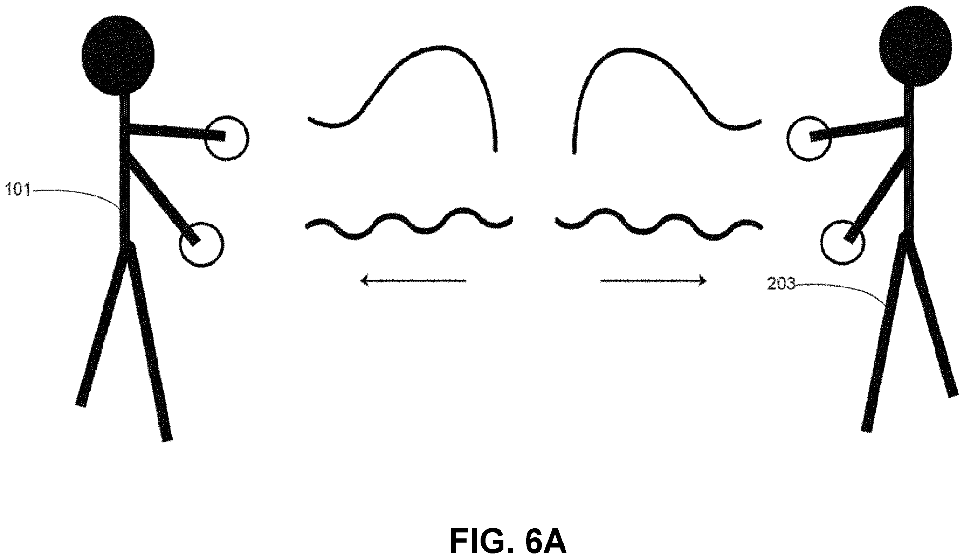

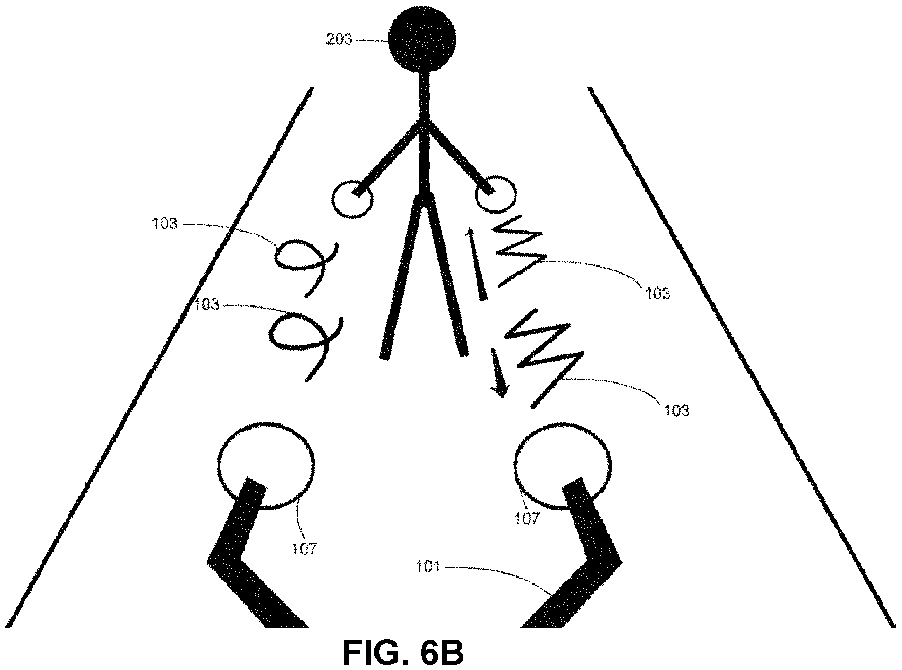

This may be followed by one or more phases of more advanced passive-mirroring gameplay wherein the system controls the display components to incrementally move the avatar to a position in which the avatar is facing the student within the scene, as illustrated by FIG. 6A. For sake of greater clarity, FIG. 6B shows the same scene as FIG. 6A, but from the student's first-person perspective. Again, the avatar also may be increasingly made to look more realistic as gameplay advances.

Yet further, referring to FIG. 7A, during the various phases within the passive-mirroring gameplay mode, the system may be configured to gradually decrease the visibility of the movement targets 105, and/or of the movement indicators 103 by changing the indicator time window for the student 101 as gameplay advances so that the student sees less and less of what is coming ahead. This leads toward the direct-mirroring mode of gameplay (described in more detail below) because the student may be compelled to start watching the partner's movement indicator(s) for visual cues as to how to move. FIG. 7A shows the indicator time window changed in default mode, whereas FIG. 7B shows the indicator time window changed in inverted mode. It should be understood that the indicator time windows, Time A, and Time B depicted in FIGS. 7A and 7B are provided for explanatory purposes and would not actually appear in the rendering of the virtual scene.

Finally, play switches to the direct-mirroring mode, in which no movement indicators are shown for the student or the therapist, such that the student must follow (by mirroring) the partner's hands directly to succeed at gameplay.

The system may be configured to provide `success feedback' to the student and/or therapist, consisting of, e.g., haptic (tactile), visual, and/or auditory feedback, as the student successfully follows each portion of the movement path or partner's movements. The goal is to gradually lead the student into mirroring the partner's movements directly and intentionally. In some embodiments, the therapist's movement guides 103 and/or movement targets 105 also may be dimmed or shortened simultaneously with the student's (not illustrated in FIGS. 7A and 7B).

Thus, in the final, direct-mirroring mode of gameplay illustrated by FIG. 8, there will no longer be any movement indicators 103 for the student 101 to follow, and no movement indicators for the partner avatar, but, instead, the student must follow (or mirror) the partner avatar's 203 hand motions. Simultaneously, in the final, direct-mirroring mode of play, the system may configure the avatar to look like a realistic depiction of the therapist (or other trusted person in the student's life). In AR and MR systems, the system may be configured to show the actual therapist, rather than an avatar. In fact, in certain AR and MR embodiments, the system may be configured to incrementally eliminate or decrease virtual elements of the therapist's representation in the XR environment thereby slowly revealing the actual therapist until the student sees no virtual elements associated with the therapist, but instead sees the actual therapist without any virtual elements.

In some embodiments, in this direct-mirroring mode of play, the partner avatar may be facing the student and initiate movements, which generate delayed movement indicators for the student to follow, i.e., the aforementioned live-type movement path. Thus, the student is mirroring the partner with a delay (sometimes referred to as a "preceding time" hereinbelow). Over time, the therapist may incrementally reduce the delay, and reduce the visual cueing provided by the movement indicators 103, until the student is mirroring the partner avatar by directly watching and following the partner avatar's movements. This mode is useful for a therapist to be initiating and leading the movements in real-time, to engage in mirroring activity that more closely resembles what happens in real-life mirroring interventions.

In some embodiments, in order to better motivate the student/player and to provide positive feedback, the system may measure the performance of the student (i.e., his success in closely tracking the patterns, or mirroring the partner avatar's motions), called `follow success', and provide a score for each game segment, such as shown at 109 in FIG. 1.

For instance, follow success may be measured by the system by evaluating a movement indicator to be successfully followed by the student when the student aligns his/her follow marker in space with the appropriate movement target 105 within a specified tolerance, e.g., as a function of the distance between the center of the student's follow marker 107 and the visual boundary of the movement target 105. Different tolerances may be applied to the evaluation of success. The accuracy may be used in providing scaled success feedback.

The follow successes of all the movement indicators presented during the course of a gameplay level may be aggregated into a collection of values and presented as a level success score. There also may be overall scores for aggregating statistics about how the student performed across all movement indicators. There also may be scores that hold statistics about how the student performed in particular ways, for example, how well the student performed with each body part or how well the student performed at following movement indicators as they moved in particular ways to train particular types of movements, such as crossing the student's midline or any other particular movement-type that may be help for the therapist to analyze.

The system may provide continuous and/or discrete success feedback (for example visual, haptic, auditory, scoring, or some combination thereof) to indicate to the student how well the student is following each movement indicator. A final score may be shown at the end of a session based on the success during the session.

Based on user/operator preference, the partner avatar's gameplay may be selectively set to either an automatic setting, in which the software controls the avatar (e.g., via an artificial intelligent bot), or a manual setting, in which the therapist controls the avatar, such as by having a second XR headset, sensors, etc. (hereinafter "XR rig") and "playing the game" via the second XR rig in a two (or more) player networked environment. The therapist may engage in any conversation with the student that is deemed helpful.

As previously noted, the avatar may be chosen to meet the student's needs for social comfort during any each session. When, eventually, a realistic human avatar is displayed, it will typically be the final use of mirroring training before practicing the same skills in real life with the therapist. In some embodiments, an artificial intelligence bot may be configured to offer encouragement to the student, just as an actual therapist might do when the partner avatar is being controlled by the therapist.

The system may be configured to control speakers (headphones) in the headset for playing music, sound effects, and/or other auditory output, including possible gameplay cues, as part of the XR experience in order to make the training more pleasurable and/or exciting. The music may be synchronized to the visual aspects of the XR experience, e.g., the movement indicators change color as the dominant musical instrument changes, the size of the motions increase with the primary pitch of the music, the movement indicators speed up or slow down with the tempo or volume of the music, the music may crescendo as one completes a difficult portion of gameplay, the movement indicators may be designed to cause the player to mimic the motions that an orchestral conductor might make to conduct the music that is being heard, etc. The movement indicators may be configured to lead the player to follow a path via movements that are typically flowing and 'lyrical'. The music may be synchronized with the movement indicators such that they create a sense in the user of dancing or moving to the music as the user follows them. The synchrony with the music may be created at least in part by computer algorithm via musical analysis in the case of movement indicators.

The system may be configured to control the display to render a second type of movement guide indicator interspersed within the flowing lines movement indicators that are of a more percussive nature and that lead the student to perform more forward/backward type motions. Percussive-type movement guide indicators lead the player to more rhythmic movements, such as smashing objects, e.g., to emphasize a rhythmic phrase in the music or a sound effect. Thus, in an embodiment, in addition to the flowing lines type movement indicators, the system may control the display and audio components to further include special "percussive" movement indicators e.g., balls or rotating cubes. The percussive movement indicators may appear within the flowing line type movement indicators 103. The student must "smash" the percussive movement indicators when they arrive at the movement targets 105 by rapidly moving a hand through the percussive movement indicator as it approaches., e.g., making some quick motion, such as a slicing, hammering, or punching motion. The system may control the audio components of the system so that these percussive guide indicators are accompanied by a particular rhythmic phrasing within the music or an explosive sound effect to make it more entertaining. In some embodiments, the system may control the audio components of the system to generate a particular musical phrase or sound effect if the student successfully "smashes" the percussive movement indicator. In some embodiments, the system may be configured to control the display components of the system to cause the visual presentation of the virtual image of student's hands to be temporarily altered (e.g., turned into a sphere, hammer, boxing glove. etc., or may be increased in size) just before the percussive movement indicator must be "smashed" in order to serve as a visual cue to perform a "smash". In some embodiments, additionally or alternately, the system may be configured to control the audio components of the system to generate an auditory cue to perform a "smash", such as a particular sound effect. In some embodiments, different types of percussive guides may be provided along with real-time movement dynamics analysis in order to expand the type of movements. For instance, different visual representations of percussive guide indicators may correspond to different types of percussive motions that the student must perform. For instance, a cube might correspond to a punching motion that must be performed, whereas a sphere might correspond to a dabbing type of motion that must be performed, and a pyramid might correspond to a slashing type of motion that must be performed. The visual appearance and dynamic behavior of each percussive movement guide indicator may be tailored to evoke the targeted movement type.

In some embodiments, the system may be configured to control the display components of the system to cause the percussive movement indicators to appear separate from the flowing line type movement indicators 103.

Students will be motivated to participate and succeed because it is a fun game that gives visual, auditory, tactile, and score-based feedback as they play. The system may be used in special education programs at schools, for home-use following therapist recommendation, and at private clinics serving the same population as special education programs.

The student may receive a real-time score during gameplay, wherein the score relates to how well the player has 'caught' the music by following and/or smashing the various movement indicator.

In some embodiments, the training can be undertaken simultaneously with multiple students, each with his/her own XR rig and personal avatar arranged separately from each other in the same virtual space. The actual students may be in the same physical space or may be joining the shared XR experience remotely from different locations over a computer network.

In some embodiments, the XR headset may include cameras within the headset to observe the student's eyes and eye tracking software to track where the student is looking. This data can be used to analyze the student's capabilities with regard to looking the avatar in the eye. In some embodiments, the student may be instructed where to look (e.g., at the avatar or in the avatar's eyes) and scored on such performance. For instance, in some embodiments, the student might be instructed to look the avatar in the eyes or to look at one of the avatar's hands as the student is mirroring the avatar. The system might be configured to insert visual and/or auditory clues into the presentation to indicate the need to do this, and/or to include success feedback of different types when it is done correctly, including scoring.

In some embodiments, the threshold for success (and thus for generating success feedback) may be dynamic, e.g., based on the student's recent history of being able to keep his/her eyes where instructed. In an example, success may first be defined so that, at first just a split second of holding gaze yields success, but the time required to hold the avatar's gaze to yield success is dynamically increased over time in order to build toward longer gazing.

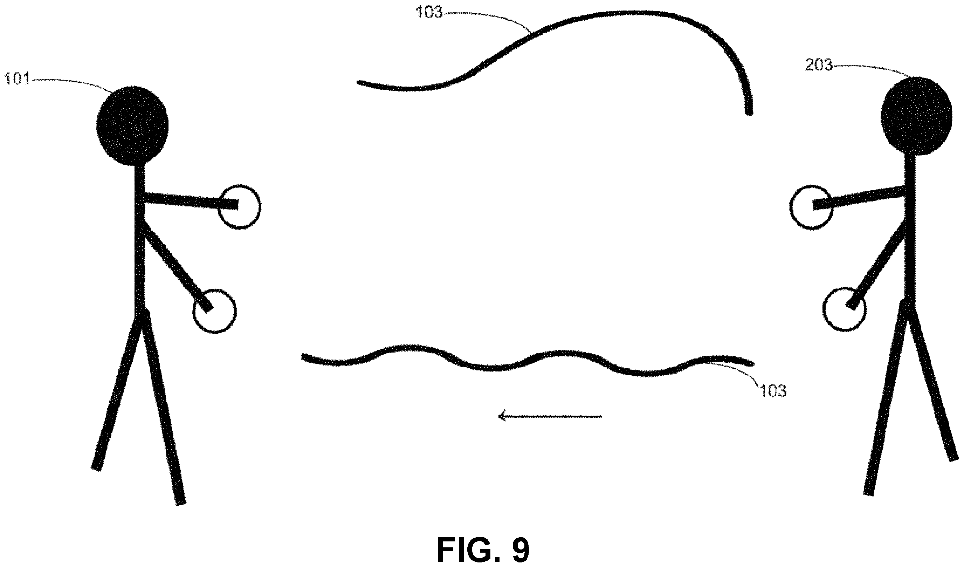

In some embodiments, such as illustrated by FIG. 9, there may be a mode of direct-mirroring game play in which the partner avatar 203 (either bot-controlled or directly controlled by the therapist) moves his/her body parts and the system generates movement indicators 103 that track the movements and flow from the therapist avatar 203 toward the student 101, which the student must "catch" (i.e., follow).

The flowing line type movement indicators 103 discussed and shown so far in this disclosure work very well for leading players into following paths in the frontal plane (up/down and left/right), but may not work as well for leading players to follow longitudinally (forwards/backwards). They are also limited in how well they can guide players to closely follow movements with varying speed. It is important to lead the player through a complete range of motion and through a range of movement dynamics to reach higher degrees of attention and sensory regulation required for optimal development of mirroring and learning skills.

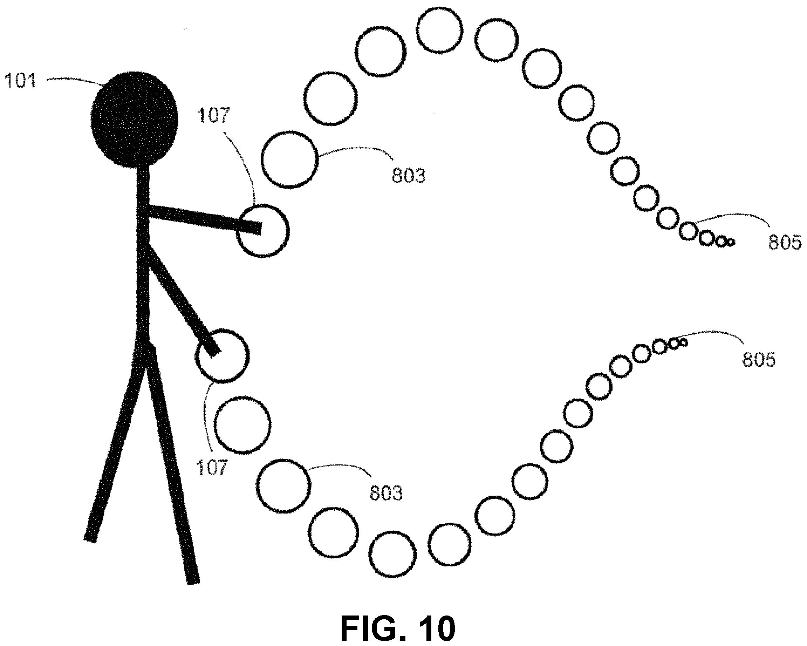

Thus, in some embodiments, such as illustrated by FIG. 10, the movement indicator may take the form of a sphere 803 or other object (for example, a hand or airplane) that moves through the virtual space, wherein the student 101 must follow its motion by aligning the appropriate follow marker 107 with the sphere 803. The sphere indicator 803 may also show the student where and how fast the sphere will be moving in the near future (over the indicator time window) by extending some sort of visual sign in the direction of the upcoming movement. For instance, a series of smaller secondary orbs that move out along a line from the main orb, with each secondary orb getting smaller as it shows the indicated movement further into the future, such as illustrated at 805 in FIG. 10. For example, if a user is meant to follow the sphere with a hand, when the user is meant to keep their hand in one place, the system may generate a display that shows the main sphere 803. If the user is meant to soon move their hand to the left, the system will generate a display that shows smaller spheres moving out to the left of the main sphere in anticipation of the movement. The faster the upcoming movement, the faster and further out the smaller orbs will move, acting as an indication of upcoming movement speed as well as direction. The visual characteristics of the object that is to be followed by the student may be chosen by the student or the therapist from a number of choices within the system or may be input by the student or therapist.

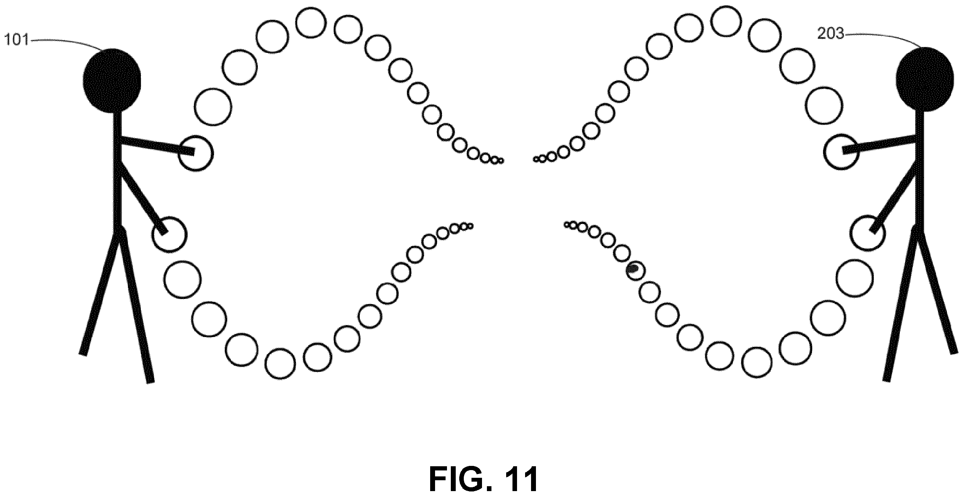

In some embodiments, such as illustrated by FIG. 11, both the therapist and the student may play using sphere indicators simultaneously in a semi-mirroring mode of gameplay.
In other embodiments, the systems, methods, and apparatus discussed herein may be used to aid students with proprioception. Proprioception is the facility of knowing where one's body parts are located in space (and/or knowing the amount of force being applied by a body part). In particular, certain individuals have impairment of their proprioception facility, often due to illness, injury, or genetics. In an embodiment, the follow marker(s) (see e.g., reference numeral 107 in FIGS. 1 and 7) may be turned off so that the student does not see a representation of their hand(s) (or other body part(s) as the case may be) that are trying to track the movement indicator 103. In this type of mode of operation/embodiment, the student must rely much more heavily on their own proprioception facility to track the movement indicator(s), thereby training the student in proprioception.

### Specific Examples

In one specific exemplary embodiment, the student may either stand or sit while wearing the audio/video XR headset while holding a controller in each hand.

The therapist, while supervising, can make choices about the direction of the game play using any suitable user interface device, such as a physical remote-control device, a remote-control app loaded onto a cellular telephone or tablet computing device, etc.

The therapist or Al bot will lead the student through a series of sessions from solo gameplay into directly facing and mirroring a human-like partner player represented by an avatar. The sessions may be performed over a period of time (including, but not limited to, hours, days, weeks, months, years, lifetime).

The order of the playing of gameplay levels and application of steps may be determined in different ways. In one embodiment, the system may present a predetermined set of gameplay levels for playing and may apply one or more steps between them in a predetermined manner, as preconfigured within the system.

In embodiments, the system may present a variable number of gameplay levels for playing and may apply a variable number of steps between each gameplay level as the user progresses through the training. After a first predetermined gameplay level is played, the number, type, and difficulty of subsequent gameplay levels to include in the set may be determined by the system based on the success of the student in playing the preceding gameplay level(s) (e.g., as determined by the student's score). For instance, If the student achieves a score (whether single or multiple aggregate or individual values thereof) above one or more predetermined thresholds, the system will change to a new gameplay level, possibly after applying one or more steps. Otherwise, it may repeat the same gameplay level again or switch to a different gameplay level of similar or lesser difficulty to let the student try again. The choice of thresholds and steps to apply between each gameplay level may be predetermined by the system.

In another embodiment, the order of playing gameplay levels may be as described in connection with the previous embodiment, except that the therapist makes the choices at one or more of the points where the system otherwise would make choices about which gameplay level to play, what score thresholds to use, and which steps to apply between the gameplay levels. The therapist may make these choices during a session with the student (in-person or remotely over a computer network) as the need to make a choice arises; or may make these choices ahead of time and lay out a predetermined set of choices for the system to follow as the student progresses, whether the instructor is in-person with the student or working remotely and possibly at a different time, or a combination of both of these options.

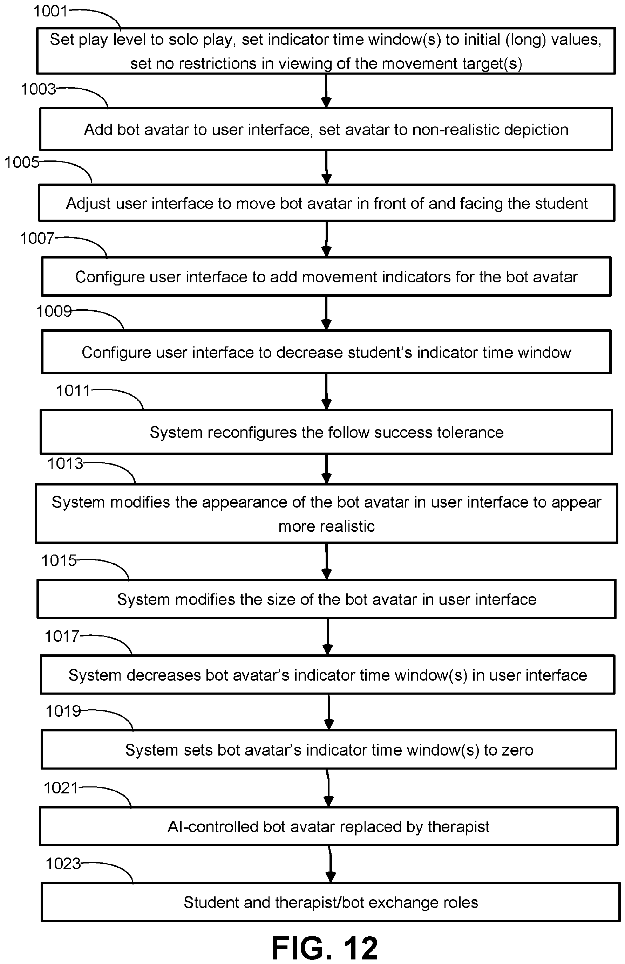

FIG. 12 is a flowchart illustrating one possible treatment course for purposes of example. At step 1001, the system presents the student with a user interface for solo gameplay. The indicator time window(s) are set to initial values with no restriction of viewing of the movement target 105, and without a bot or therapist in the virtual scene. This is the acclimation step, for the student to learn how to play gameplay levels and gain skill at following the movement indicators 103.

At step 1003, the system configures the user interface to add a bot avatar 203 (e.g., Al-controlled). The bot avatar is positioned somewhere in the scene where the student can readily see it, but not directly in front of the student. The bot avatar will generally start as something that is familiar and comfortable for the student, perhaps a favorite animal or cartoon character. The therapeutic goal of this step is for the student to get used to the partner avatar's presence and participation.

In step 1005, the system configures the III to move the bot avatar 203 directly in front of the student and facing the student. This may be performed in one step or may be performed incrementally over a plurality of sessions, or it may occur incrementally during one continuous game play session (e.g., the bot avatar takes a step or two at predetermined intervals between the student playing gameplay levels). In an embodiment, the distance from the student may be adjusted by the therapist (or Al) to assure it is comfortable for the student. In an embodiment, the distance between the student and the bot avatar also may be lessened in steps over time.

In step 1007, the system adds movement indicators 103 for the bot avatar to the user interface. The bot avatar begins to follow its own copy of the gameplay level's movement indicators(s). If the bot avatar is facing the student, or close to facing the student, the movement path(s) will be mirrored across the bot avatar's sagittal plane, so that, for example, as the student follows a movement path with his/her right hand, the bot avatar follows its copy of the path with the left, in a mirror-image manner. For example, with the flowing lines type indicator option, a second set of lines will flow towards the bot avatar that the bot avatar will follow. In the sphere indicator type of movement indicator option, the student and the bot avatar each have sphere indicators in front of them to follow.

In some embodiments, initially, the bot avatar's copy of the movement indicator(s) may be played earlier in time than those of the student by a length of time herein termed the preceding time window. Thus, the bot avatar moves in a way that precedes the movement of the student. The music, if playing, may be configured to be in sync with the timing of the student's movement indicators. The preceding time window may be set by the system or by the therapist.

Next as part of step 1007, the preceding time window may be made shorter, so the bot avatar moves closer in time synchronization with the student. That is, the bot's movements precede the student's movements by a shorter time.

Next as part of step 1007, the preceding time window may be set to zero, so that the bot avatar moves in synchronization with the student, and the movement indicator(s) shown for the bot avatar moves in synchronization with the student's' movement indicator(s).

In step 1009, the system starts to shorten the student's indicator time window(s), whether in default or inverted mode. That is, the user interface shows the student less indication as to what moves the student will be making next. As the system shortens the indicator time window(s), the system still tracks (measures and stores in memory) the follow success, and provides success feedback to the student (e.g., a score that is displayed in the UI, and/or haptic feedback), even when there may no longer any movement target 105 being shown to the student (in the case of an indicator time window in Inverted mode), by means of the system tracking the spatial relation between the student's follow marker 107 and the student's movement target 105 position along the movement path, even though it is not shown. As the indicator time window shrinks, the student has less indication of what is expected next of them and is forced to look more to the bot avatar (whose indicator time window is not changed in this step) to get an indication of what movements are coming next. The student will be motivated to start to watch the bot avatar's movement indicator(s) for help determining what is coming next. This process will eventually become a mirroring action. Typically, this step will be applied only once the preceding time window is set to zero, but it may be applied at other times at the discretion of the therapist.

Toward the end of this step, the system sets the student's indicator time window to zero. Thus, no movement indicator and thus also no movement target for the student is displayed in the UI. The student is now required to mimic the bot avatar's movements and possibly movement indicators (if they are still shown) without the help of his/her own movement indicator at all. At this point, the student is able to follow the movements only by watching the bot avatar and mirroring it and/or by watching its movement indicators and mimicking the movements that the bot uses to follow them. Success feedback is still applied.

In step 1011, the system reconfigures the follow success tolerance to widen or narrow the range of movement that is considered a success in order to help the student adjust to changes in difficulty as different steps are applied, and to challenge the student further as he/she improves at the training.

Next, in step 1013, the bot avatar's appearance is changed to look more human and less fantastical. This step may be repeated a number of times, possibly until the bot avatar looks like a very realistic human with a full virtual body, realistic face, and realistic facial and eye movements.

Next, in step 1015, the system modifies the bot avatar to scale it to a larger size, at the discretion of the therapist. The goal of this step is to change the avatar's size over time to train the student to be comfortable with mirroring avatars of different sizes, and in particular avatar's larger than themselves.

In step 1017, the system decreases the bot avatar's indicator time window on one or more of its movement indicators. This may be done at any time but is typically done starting only after the student's indicator time window(s) has(ve) been set to zero.

Next, in step 1019, the system sets the bot avatar's indicator time window(s) to zero. When the student's corresponding indicator time window(s) is also zero, the student has to mirror the bot avatar's follow marker or corresponding body part directly, without the help of any movement indicator 103.

Next, in step 1021, the Al-controlled bot avatar is replaced by the therapist (e.g., via the therapist donning a second XR rig and joining the session). The therapist's avatar may range from fantastical to highly realistic, and generally will be the same as the Al-controlled bot avatar at the time of the switch for continuity of the student's experience. The therapist has the option to start generating live paths for the student to follow or join the training by following recorded or procedural paths along with the student in the same way that the bot avatar did. The therapist may replace the Al-controlled bot avatar for any period of time and for any number of times. The duration and frequency of the therapist's replacement of the bot avatar may be at the therapist's discretion. For example, the therapist may take the place of the bot when working with the student during a treatment session, and then the bot may be used when the student is training at home with the system outside of a treatment session. Alternately, the therapist may replace the bot only after the student has progressed to the stage of mirroring the bot directly without the help of movement indicators. After this step is applied, any other previously discussed steps involving the bot may be re-applied with the therapist instead of the bot.

Finally, in some embodiments, in step 1023, the student takes on the role of the creator of live paths, and the bot or therapist, whichever is active in the training, takes on the typical roll of the student as the follower of the live paths. Thus, the student acts much like the bot or therapist, except without decision-making ability to change operational parameters or apply steps. This step is intended to train students who have difficulty in leading other people in mirror activities. If step 1023 has been applied successfully, the student may revert back to the usual role.

### Conclusion

The afore-described methods and apparatus address a number of common challenges relating to attunement, mirroring, and social-emotional skills when working with neurodiverse populations. Its educational and therapeutic applications can provide a bridge to help neurodiverse populations overcome the most difficult challenges they face, which include but are not limited to: 1) meaningful social connections that are challenged when social anxiety and sensory motor challenges interfere; 2) difficulties for clients and families to continue therapeutic practices in-between sessions without a clinician, limiting the success of the therapy plan; and 3) social overwhelm during person-to-person interactions, leading to emotional dysregulation.

In summary, building attunement and mirroring skills is necessary to improve an ASD student's ability to make personal connections and attend in academic and social situations. While Dance Movement Therapists (DMTs) are well-trained to make connections, the inherent interpersonal nature of DMT may not always be optimal to overcome a client's challenges with person-to-person attunement and socialization. Additionally, it can be difficult to generalize DMT skills beyond therapy to academic and family settings. Technology may be the key to overcoming these challenges.

In the discussion above, the users of the system have often been referred to as the student and the therapist. However, these are merely illustrative terms and are not meant to be limiting. The therapist could be any person and the student could be any person.

Having thus described a few particular embodiments of the invention, various alterations, modifications, and improvements will readily occur to those skilled in the art. Such alterations, modifications and improvements as are made obvious by this disclosure are intended to be part of this description though not expressly stated herein, and are intended to be within the spirit and scope of the invention. Accordingly, the foregoing description is by way of example only, and not limiting. The invention is limited only as defined in the following claims and equivalents thereto.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer readable medium for execution by a computer or processor. Examples of non-transitory computer-readable storage media include, but are not limited to, a read only memory (ROM), random access memory (RAM), a register, cache memory, semiconductor memory devices, magnetic media such as internal hard disks and removable disks, magneto-optical media, and optical media such as CD-ROM disks, and digital versatile disks (DVDs).

Moreover, in the embodiments described above, processing platforms, computing systems, controllers, and other devices containing processors are noted. These devices may contain at least one Central Processing Unit ("CPU") and memory. In accordance with the practices of persons skilled in the art of computer programming, reference to acts and symbolic representations of operations or instructions may be performed by the various CPUs and memories. Such acts and operations or instructions may be referred to as being "executed," "computer executed" or "CPU executed."

One of ordinary skill in the art will appreciate that the acts and symbolically represented operations or instructions include the manipulation of electrical signals by the CPU. An electrical system represents data bits that can cause a resulting transformation or reduction of the electrical signals and the maintenance of data bits at memory locations in a memory system to thereby reconfigure or otherwise alter the CPU's operation, as well as other processing of signals. The memory locations where data bits are maintained are physical locations that have particular electrical, magnetic, optical, or organic properties corresponding to or representative of the data bits. It should be understood that the exemplary embodiments are not limited to the above-mentioned platforms or CPUs and that other platforms and CPUs may support the provided methods.

The data bits may also be maintained on a computer readable medium including magnetic disks, optical disks, and any other volatile (e.g., Random Access Memory ("RAM")) or non-volatile (e.g., Read-Only Memory ("ROM")) mass storage system readable by the CPU. The computer readable medium may include cooperating or interconnected computer readable medium, which exist exclusively on the processing system or are distributed among multiple interconnected processing systems that may be local or remote to the processing system. It is understood that the representative embodiments are not limited to the above-mentioned memories and that other platforms and memories may support the described methods.

In an illustrative embodiment, any of the operations, processes, etc. described herein may be implemented as computer-readable instructions stored on a computer-readable medium. The computer-readable instructions may be executed by a processor of a mobile unit, a network element, and/or any other computing device.

There is little distinction left between hardware and software implementations of aspects of systems. The use of hardware or software is generally (but not always, in that in certain contexts the choice between hardware and software may become significant) a design choice representing cost vs. efficiency tradeoffs. There may be various vehicles by which processes and/or systems and/or other technologies described herein may be effected (e.g., hardware, software, and/or firmware), and the preferred vehicle may vary with the context in which the processes and/or systems and/or other technologies are deployed. For example, if an implementer determines that speed and accuracy are paramount, the implementer may opt for a mainly hardware and/or firmware vehicle. If flexibility is paramount, the implementer may opt for a mainly software implementation. Alternatively, the implementer may opt for some combination of hardware, software, and/or firmware.

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples may be implemented, individually and/or collectively, by a wide range of hardware, software, firmware, or virtually any combination thereof. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Application Specific Standard Products (ASSPs); Field Programmable Gate Arrays (FPGAs) circuits, any other type of integrated circuit (IC), quantum computer, and/or a state machine.

The present disclosure is not to be limited in terms of the particular embodiments described in this application, which are intended as illustrations of various aspects. Many modifications and variations may be made without departing from its spirit and scope, as will be apparent to those skilled in the art. No element, act, or instruction used in the description of the present application should be construed as critical or essential to the invention unless explicitly provided as such. Functionally equivalent methods and apparatuses within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods or systems.

It is also to be understood that the terminology used herein is for the purpose of describing particular embodiments only, and is not intended to be limiting.

In certain representative embodiments, several portions of the subject matter described herein may be implemented via Application Specific Integrated Circuits (ASICs), Field Programmable Gate Arrays (FPGAs), digital signal processors (DSPs), and/or other integrated formats. However, those skilled in the art will recognize that some aspects of the embodiments disclosed herein, in whole or in part, may be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein may be distributed as a program product in a variety of forms, and that an illustrative embodiment of the subject matter described herein applies regardless of the particular type of signal bearing medium used to actually carry out the distribution. Examples of a signal bearing medium include, but are not limited to, the following: a recordable type medium such as a floppy disk, a hard disk drive, a CD, a DVD, a digital tape, a computer memory, etc., and a transmission type medium such as a digital and/or an analog communication medium (e.g., a fiber optic cable, a waveguide, a wired communications link, a wireless communication link, etc.).

The herein described subject matter sometimes illustrates different components contained within, or connected with, different other components. It is to be understood that such depicted architectures are merely examples, and that in fact many other architectures may be implemented which achieve the same functionality. In a conceptual sense, any arrangement of components to achieve the same functionality is effectively "associated" such that the desired functionality may be achieved. Hence, any two components herein combined to achieve a particular functionality may be seen as "associated with" each other such that the desired functionality is achieved, irrespective of architectures or intermediate components. Likewise, any two components so associated may also be viewed as being "operably connected", or "operably coupled", to each other to achieve the desired functionality, and any two components capable of being so associated may also be viewed as being "operably couplable" to each other to achieve the desired functionality. Specific examples of operably couplable include but are not limited to physically mateable and/or physically interacting components and/or wirelessly interactable and/or wirelessly interacting components and/or logically interacting and/or logically interactable components.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, where only one item is intended, the term "single" or similar language may be used. As an aid to understanding, the following appended claims and/or the descriptions herein may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"). The same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B." Further, the terms "any of" followed by a listing of a plurality of items and/or a plurality of categories of items, as used herein, are intended to include "any of," "any combination of," "any multiple of," and/or "any combination of multiples of" the items and/or the categories of items, individually or in conjunction with other items and/or other categories of items. Moreover, as used herein, the term "set" or "group" is intended to include any number of items, including zero. Additionally, as used herein, the term "number" is intended to include any number, including zero.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein may be readily broken down into a lower third, middle third and upper third, etc. As will also be understood by one skilled in the art all language such as "up to," "at least," "greater than," "less than," and the like includes the number recited and refers to ranges which can be subsequently broken down into subranges as discussed above. Finally, as will be understood by one skilled in the art, a range includes each individual member. Thus, for example, a group having 1-3 items refers to groups having 1, 2, or 3 items. Similarly, a group having 1-5 items refers to groups having 1, 2, 3, 4, or 5 items, and so forth.

Moreover, the claims should not be read as limited to the provided order or elements unless stated to that effect. In addition, use of the terms "means for" in any claim is intended to invoke 35 U.S.C. §112, 6 or means-plus-function claim format, and any claim without the terms "means for" is not so intended.

Although the invention is illustrated and described herein with reference to specific embodiments, the invention is not intended to be limited to the details shown. Rather, various modifications may be made in the details within the scope and range of equivalents of the claims and without departing from the invention.

Throughout the disclosure, one of skill understands that certain representative embodiments may be used in the alternative or in combination with other representative embodiments.

## Claims

1. A computer-readable device comprising non-transitory instructions, which, when executed by a processor, cause the processor to perform operations, the operations comprising:
generating an extended reality user interface (XRUI);
tracking movement of at least one body part of a user in space;
generating in the XRUI a follow marker, the follow marker comprising a representation of the movement of the tracked user's body part;
generating in the XRUI a movement indicator, the movement indicator comprising a representation of a curvilinear path, wherein the path is depicted over time as moving toward a predetermined plane within the XRUI;
calculating a distance in the XRUI between the follow marker and the movement indicator in the predetermined plane over time; and
determining a follow success value indicative of the ability of the user to track the movement indicator with the follow marker.

2. The computer-readable device of claim 1 wherein the operations further comprise:
generating in the XRUI an avatar of another being.

3. The computer-readable device of claim 2 wherein the operations further comprise:
gradually changing an appearance of the avatar.

4. The computer-readable device of claim 1 wherein the operations further comprise:
gradually varying the movement indicator so as to increasing a level of skill required of the user to follow the movement indicator.

5. The computer-readable device of claim 2 wherein the operations further comprise:
generating in the XRUI a supplemental movement indicator, the supplemental movement indicator comprising a mirror image of the movement indicator moving toward the avatar; and
controlling a body part of the avatar to track the supplemental movement indicator.

6. The computer-readable device of claim 1 wherein the operations further comprise:
including an audio track within the XRUI.

7. The computer-readable device of claim 6 wherein the audio track comprises music that is synchronized to the movement indicator.

8. The computer-readable device of claim 2 wherein the movement indicator is generated so as to appear to be emanating from a body part of the avatar.

9. An apparatus for training a user in social mirroring skills comprising:
an extended reality render module configured to generate an extended reality user interface (XRUI), the XRUI comprising a movement indicator, the movement indicator comprising a representation of a curvilinear path, wherein the path is depicted over time as moving toward a predetermined plane within the XRUI;
a tracking module configured to track movement of a sensor in space;
a motion module configured to generate in the XRUI a follow marker, the follow marker comprising a representation of the movement of the sensor in space; and
a data capture/scoring module configured to calculate a distance in the XRUI between the follow marker and the movement indicator in the predetermined plane over time and determine a follow success value indicative of the accuracy with which the follow marker tracked the movement indicator.

10. The apparatus of claim 9 wherein the extended reality render module further displays a score based on the follow success value and displays the score within the XRUI.

11. The apparatus of claim 9 wherein the extended reality render module generates the movement indicator by using the tracking module to track a sensor in space and generates the movement indicator by emulating the tracked movement of the sensor.

12. A method of training users in social mirroring skills, the method comprising:
generating an extended reality user interface (XRUI);
tracking movement of at least one body part of a user in space;
generating in the XRUI a follow marker, the follow marker comprising a representation of the movement of the tracked user's body part;
generating in the XRUI a movement indicator, the movement indicator comprising a representation of a curvilinear path, wherein the path is depicted over time as moving toward a predetermined plane within the XRUI;
calculating a distance in the XRUI between the follow marker and the movement indicator in the predetermined plane over time; and
determining a follow success value indicative of the ability of the user to track the movement indicator with the follow marker.

13. The method of claim 12 further comprising:
generating in the XRUI an avatar of another being.

14. The method of claim 13 further comprising:
gradually changing a location of the avatar within the XRUI from a position that is off to a side of the user to a position in front of the user.

15. The method of claim 13 further comprising:
generating in the XRUI a supplemental movement indicator, the supplemental movement indicator comprising a mirror image of the movement indicator moving toward the avatar; and
controlling a body part of the avatar to track the supplemental movement indicator.
